(19) Europäisches Patentamt European Patent Office Office européen des brevets

(11) **EP 4 589 539 A1**

(12) **EUROPEAN PATENT APPLICATION**

(43) Date of publication:
**23.07.2025 Bulletin 2025/30**

(21) Application number: **25151991.4**

(22) Date of filing: **15.01.2025**

(51) International Patent Classification (IPC):
**G06T 11/00** (2006.01)

(52) Cooperative Patent Classification (CPC):
**G06T 11/006**; G06T 2211/424; G06T 2211/432

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.01.2024 US 202463623069 P**

(71) Applicant: **Carl Zeiss X-Ray Microscopy, Inc.**
**Dublin, California 94568 (US)**

(72) Inventors:
• **Yang, Faguo**
  **Dublin, 94568 (US)**
• **Andreyev, Andriy**
  **Dublin, 94568 (US)**
• **Andrew, Matthew**
  **Dublin, 94568 (US)**

(74) Representative: **Gulde & Partner**
**Patent- und Rechtsanwaltskanzlei mbB**
**Berliner Freiheit 2**
**10785 Berlin (DE)**

(54) **IMAGE RECONSTRUCTION FOR WIDE FIELD MODE CT SCANS**

(57) A method and system for reconstructing three-dimensional volumes from truncated X-ray projections acquired in a single rotation wide field mode (SRWFM) computed tomography (CT) scan offsets the sample's rotation axis relative to the X-ray propagation optical axis, the effective field of view is thus enlarged beyond the detector's nominal dimensions. Redundancy weighting is applied to compensate for incomplete angular sampling in truncated projections, performing this weighting after ramp filtering and prior to back-projection in an analytical (e.g., filtered back projection) or iterative reconstruction algorithm. Additional corrections include adjusting pixel intensities based on the angle between the X-ray optical axis and the detector plane, as well as remapping projection data onto a virtual detector plane aligned with the rotation axis. These techniques mitigate image artifacts, reduce noise, and ensure accurate attenuation coefficient representation, ultimately enabling high-quality reconstructions of larger or asymmetrically positioned samples from a single 360-degree rotation.

Fig. 2A

EP 4 589 539 A1

Rotation Stage

detector

110

OA (of 104)

102

124

X-ray source

# Fig. 2B

# Projection of Rotation Axis

y

124

$\sigma$     $\sigma$

124-E

X

RA

# Fig. 6

**Description**

BACKGROUND OF THE INVENTION

X-ray micro tomography systems provide high-resolution, non-destructive imaging of internal structures in samples. The systems are utilized in a variety of industrial and research applications, such as mining, manufacturing, materials science, clinical research, and failure analysis, in examples. The systems provide the ability to visualize features in samples without the need to cut and slice the samples.

X-ray micro tomography systems include X-ray projection systems that produce projection data of the sample and computer systems that reconstruct tomographic volumes of the sample from the projection data. In operation, the X-ray projection system executes a scan of a sample at different projection angles to produce the projection data. During a scan, X-rays are directed to the sample, and are absorbed or scattered by the sample as the X-rays travel through the sample. The X-rays not absorbed or scattered away are transmitted through and modulated by the sample. A detector system receives the transmitted X-rays, and creates an image representation, in pixels, of the received X-rays. The series of X-ray projections at different angles produced from the scan form the projection data of the sample. Then, the computer system accesses the projection data, and applies tomographic reconstruction algorithms to the projection data to reconstruct volume datasets of the sample. A volume dataset is a three-dimensional (3D) representation of the entire sample, and a slice is a two dimensional (2D) cross-sectional image of the sample based on the volume dataset.

The most commonly used reconstruction algorithms fall into a class of reconstruction techniques termed analytical reconstruction. The objective is to find a closed-form solution to the problem of reconstructing an object's internal structure from its projections. The most common analytical method is filtered back projection (FBP). The projections are first processed using a high-pass filter, usually a ramp filter, in the frequency or space domain. Then, each filtered projection is "smeared" back onto the imaging plane as if each data point emits back uniformly in the shape of the original beam. These back projections from all angles are summed up to produce the reconstructed volume, which approximates the object's internal structure. The filtering and back projection operations collectively help in obtaining a more accurate and less blurred reconstruction of the original object. One type of filtered back projection is FDK reconstruction algorithm. It is used often with X-ray micro tomography systems since it reduces artifacts associated with the typical cone-shaped beam. See Feldkamp, L.A., Davis, L.C. and Kress, J.W.

(1984) Practical Cone-Beam Algorithm. Journal of the Optical Society of America A, 1, 612-619.

Iterative reconstruction is another approach that reconstructs slices from successive estimates of the projection data by forward projecting the estimated reconstruction volume. Multiple iterations of the estimated projection data for each view angle are executed for this purpose. During each iteration, the estimated projection data are compared to the actual (e.g. measured) projection data. The result of each comparison is used to make corrections to the current estimation of the reconstructed volume, thereby creating a new estimate of the reconstructed volume. When the estimated and measured projection data are close enough, which means the iteration process converges, a volume dataset is reconstructed from the measured projection data. Exemplary iterative reconstruction algorithms include algebraic reconstruction technique (ART), simultaneous reconstruction technique (SIRT) and iterative least-squares technique (ILST). The algorithms typically differ in the way the measured and estimated projection data are compared and the kind of correction applied to the current estimate. See Lin, Q., Andrew, M., Thompson, W., Blunt, M.J. and Bijeljic, B., Optimization of image quality and acquisition time for lab-based X-ray microtomography using an iterative reconstruction algorithm, Advances in Water Resources 115, 112-124 (2018).

Analytical reconstruction and iterative reconstruction methods have advantages and disadvantages. FBP, for example, provides good image quality with relatively low processing overhead, which increases throughput and reduces cost. A disadvantage of FBP is susceptibility to image noise in the projection data. Advantages of iterative reconstruction include generally improved image quality, less susceptibility to image noise, reduced image artifacts, and the ability to reconstruct an optimal image in the case of incomplete projection data. Disadvantages of iterative reconstruction include complexity associated with selection of regularization parameters applied to the iterative reconstruction algorithm, longer processing time, and increased computational cost.

High end x-ray micro tomography (microscopy) systems (XRM) can reach a resolution of 0.5 Dm, which results in a relatively small field of view, since the number of pixels is usually fixed (e.g. 1024 by 1024). Currently many commercial XRM systems can have a wide field mode (WFM) option that tries to extend the field of view of the system beyond the detector size. In one conventional arrangement, the WFM scan mode could provide a nearly 2 times larger field of view than a regular scan. In a regular scan, the rotation axis is projected to the detector center. In one of the current WFM realizations, it is necessary to perform two scans, one is to first shift the rotation axis to the right of the detector and then rotate with the sample mounted to acquire a scan; the other one is to shift the rotation axis to the left of the detector and then acquire another scan of the sample. After the two scans, the raw projection data from the two scans are stitched together to form projections that are much larger than the detector size (e.g. for 1024 by 1024 pixel detector, the stitched projection size could be 1906 by 1906 pixels). The stitched datasets from the two rotation-axis-shifted scans are treated in FDK reconstruction as a regular scan that the rotation axis projected to detector center. One drawback, however, is that this two-scan-WFM method requires very accurate mechanical calibration to avoid image artifacts. Another method to perform WFM mode is to only shift the rotation axis once and perform a scan, which is commonly referred to as a single rotation wide field mode (SRWFM). Please note, for rotation axis shifted mode, the sample just rotates around this shifted-rotation-axis; for detector-offset systems, the detectors will keep the same offset for all projections. In general, it needs full 360-degree of scans to acquire sufficient raw data to perform the 3D volume reconstruction.

In Cho, P.S., Rudd, A.D. and Johnson, R.H., Cone-beam CT from width-truncated projections, Computerized Medical Imaging and Graphics, 20, 49-57 (1996), cone-beam CT techniques are described that permit reconstruction from width-truncated projections.

SUMMARY OF THE INVENTION

The present invention concerns analytical reconstruction and iterative reconstruction for micro-tomographic X-ray scans, so called single rotation wide field mode (SRWFM) scans with cone beam X-ray computed tomography.

A SRWFM mode is used. It involves shifting the rotation axis possibly once, but could be more, and performing a scan or scans, which can be reconstructed using the proposed reconstruction methods as described later. An imaging configuration is employed in which the axis of rotation of the sample and/or the detector are offset from the center of the system's X-ray beam. This allows for the imaging of larger samples (wide field). A set of X-ray projections from different angles are acquired by rotating the sample such as only a single time over a 360-degree angular range. For SRWFM, the sample just rotates around this shifted-rotation-axis; for detector-offset systems, the detectors will keep the same offset for all projections. In general, it needs full 360-degree of scans to acquire sufficient raw data to perform the 3D volume reconstruction. Importantly, a reconstruction approach is applied that weights different regions of the projection data in order to improve the reconstructions and reduce artifacts.

In general, according to one aspect, the invention features a method for reconstructing a three-dimensional volume of a sample from projection data obtained by an X-ray-microscopy tomographic scan. The method comprises acquiring, by a detector, X-ray projections of the sample by rotating the sample about a rotation axis that is offset with respect to an X-ray propagation axis and processing the acquired projections to account for the offset rotation axis by applying redundancy weighting to correct for incomplete angular sampling in regions of the projection data, wherein the redundancy weighting is performed before back-projection in an analytical reconstruction or before an equivalent back-projection operation in an iterative reconstruction.

Preferably, prior to the redundancy weighting, a ramp filter is applied to the projection data, wherein the redundancy weighting is performed on the ramp-filtered data.

The redundancy weighting can be applied in a region around a projection of the rotation axis on the detector and possibly employs a position-dependent weighting function that corrects for projections without requiring multiple scans.

The projection data can be adjusted by scaling pixel intensities according to an angle formed between an optical axis of the X-ray beam and a plane of the detector, preferably according to an angle formed between a plane of the detector and a virtual detector plane that is normal to a vector passing through the rotation axis and the center of the source, the scaling correcting for geometric distortions introduced by the offset axis configuration.

In a current example, the projections are remapped onto a virtual detector plane perpendicular to a line connecting the X-ray source and the rotation axis, thereby ensuring correct attenuation coefficient representation in the reconstructed volume.

The iterative reconstruction approach can include applying a noise model-based weighting to the difference between measured and forward-projected data, and subsequently incorporating the redundancy weighting before back-projection, thus improving noise handling and artifact reduction.

Typically, the projections are acquired over a full 360-degree rotation of the sample to ensure sufficient sampling of regions within the enlarged field of view and the projections may be acquired only over a single full 360-degree rotation.

In general, according to one aspect, the invention features a system for reconstructing a three-dimensional volume of a sample from projection data obtained by a single rotation wide field mode (SRWFM) X-ray scan. The system comprises an X-ray source configured to generate an X-ray beam, a rotation stage configured to hold and rotate the sample about a rotation axis offset with respect to an X-ray propagation axis, thereby providing X-ray projections with an enlarged effective field of view beyond nominal detector dimensions and a detector configured to receive said X-ray projections of the sample. A processing system configured to receive projection data from the detector, apply redundancy weighting to correct for incomplete angular sampling in regions of the projection data, wherein the redundancy weighting is performed before back-projection in an analytical reconstruction or before an equivalent back-projection operation in an iterative reconstruction. A three-dimensional volume of the sample is then reconstructed from the projection data based on the applied redundancy weighting.

The processing system is preferably further configured to apply a ramp filter to the projection data prior to applying the redundancy weighting, such that the redundancy weighting is performed on the ramp-filtered data. Also, the processing system can be configured to apply the redundancy weighting in a region around a projection of the rotation axis on the detector.

The processing system is configured to employ a position-dependent weighting function that corrects for projections without requiring multiple scans, preferably. The processing system is further preferably configured to adjust the projection data by scaling pixel intensities according to an angle formed between an optical axis of the X-ray beam and a plane of the detector, said scaling correcting for geometric distortions introduced by the offset axis configuration.

The processing system can be configured to remap the projections onto a virtual detector plane perpendicular to a line connecting the X-ray source and the rotation axis, thereby ensuring correct attenuation coefficient representation in the reconstructed volume.

In one example, the processing system is configured to execute an iterative reconstruction algorithm that includes applying a noise model-based weighting to differences between measured and forward-projected data, and subsequently incorporating the redundancy weighting before back-projection, thereby improving noise handling and artifact reduction.

A controller is configured to operate the rotation stage and acquire projections over a full 360-degree rotation, and possibly only over a single rotation, of the sample to ensure sufficient sampling of regions within the enlarged field of view.

The above and other features of the invention including various novel details of construction and combinations of parts, and other advantages, will now be more particularly described with reference to the accompanying drawings and pointed out in the claims. It will be understood that the particular method and device embodying the invention are shown by way of illustration and not as a limitation of the invention. The principles and features of this invention may be employed in various and numerous embodiments without departing from the scope of the invention.

BRIEF DESCRIPTION OF THE DRAWINGS

In the accompanying drawings, reference characters refer to the same parts throughout the different views. The drawings are not necessarily to scale; emphasis has instead been placed upon illustrating the principles of the invention. Of the drawings:

Fig. 1 is a schematic diagram of an X-ray micro tomography system to which the methods of the present invention are applicable;

Figs. 2A and 2B are schematic top views of the hardware configuration for single rotation wide field mode (SRWFM) scans showing the detector is shifted to one side (Fig. 2A) and the rotation stage is shifted to one side of the detector (Fig. 2B) with respect to the X-ray propagation axis;

Figs. 3A and 3B are slices exhibiting image artifacts for SRWFM scans with FDK reconstruction (Fig. 3A) and with iterative reconstruction (Fig. 3B);

Fig. 4 is a flow diagram showing a method based on FDK filtered back projection reconstruction of a SRWFM scan;

Fig. 5 is a top view of a rotation axis shifted SRWFM hardware configuration where the virtual detector plane is at an angle to the "original" X-ray beam propagation axis;

Fig. 6 shows the two-dimensional extent of the detector on which the rotation axis of the rotation stage is projected;

Fig. 7 is a flow diagram showing a method for iterative reconstruction for SRWFM scans; and

Figs. 8A and 8B are slices from FDK reconstruction detailed in Fig. 4 (Fig. 8A) and iterative reconstruction detailed in Fig. 7 (Fig. 8B).

DETAILED DESCRIPTION OF THE PREFERRED EMBODIMENTS

The invention now will be described more fully hereinafter with reference to the accompanying drawings, in which illustrative embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art.

As used herein, the term "and/or" includes any and all combinations of one or more of the associated listed items. Further, the singular forms and the articles "a", "an" and "the" are intended to include the plural forms as well, unless expressly stated otherwise. It will be further understood that the terms: includes, comprises, including and/or comprising, when used in this specification, specify the presence of stated features, integers, steps, operations, elements, and/or components, but do not preclude the presence or addition of one or more other features, integers, steps, operations, elements, components, and/or groups thereof. Further, it will be understood that when an element, including component or subsystem, is referred to and/or shown as being connected or coupled to another element, it can be directly connected or coupled to the other element or intervening elements may be present.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

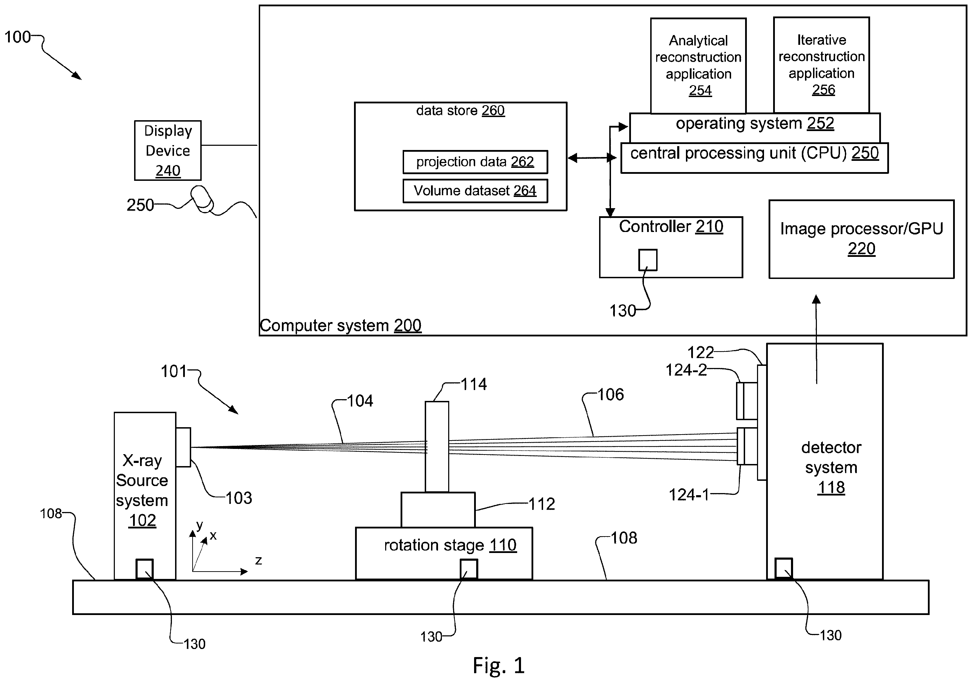

Fig. 1 is a schematic diagram of an X-ray micro tomography system 100 to which the methods and workflows of the present invention are applicable.

In general, the X-ray micro tomography system 100 combines an X-ray microscopy system 101 and a computer system 200 for receiving projections of a sample 114 and calculating volume datasets 264 from those projections.

The X-ray microscopy system 101 includes an X-ray source system 102 that generates a typically polychromatic X-ray beam 104. A rotation stage 110 has a sample holder 112 for holding the sample 114. The rotation stage 110 rotates the sample 114 in the X-ray beam 104 from the X-ray source system 102. Images or X-ray projections are captured by a detector system 118. The X-ray source system 102, the rotation stage 110, and the detector system 118 are mounted to a base 108 of the X-ray CT system 100. The computer system 200 typically receives and processes these projections and provides general control of the system 100. The computer system 200 or another computer will typically perform tomographic reconstruction using the X-ray projections to create volume datasets of the sample 114.

The X-ray source 102, in one example, is a cone-beam, polychromatic X-ray source. The polychromatic X-ray source is preferably a laboratory X-ray source because of its ubiquity and relatively low cost. Nonetheless, synchrotron sources or accelerator-based sources are other alternatives.

Common laboratory X-ray sources include an X-ray tube, in which electrons are accelerated in a vacuum by an electric field and shot into a target piece of metal, with X-rays being emitted as the electrons decelerate in the metal.

In one example, the X-ray source 102 is a rotating anode reflective type target or micro-focused source, with a Tungsten target. Targets that include Molybdenum, Gold, Platinum, Silver or Copper also can be employed. In one embodiment, a transmissive-type target configuration of the X-ray source 102 is used in which the electron beam strikes the thin target 103 from its backside. The X-rays emitted from the other side of the target 103 are then used as the beam 104.

When the sample 114 is exposed to the X-ray beam 104, the X-ray photons transmitted through the sample form an attenuated X-ray beam 106 that is received by the detector system 118. In some other examples, an objective lens such as a zone plate lens is used to form an image onto the detector system 118 of the X-ray imaging system 100.

In one embodiment of the detector system 118, a magnified projection image of the sample 114 is formed on the detector system 118 with a geometrical magnification that is equal to the inverse ratio of the source-to-sample distance and the source-to-detector distance. Generally, the geometrical magnification provided by the X-ray stage is between 2x and 100x, or more. In this case, the resolution of the X-ray image is limited by the focus spot size or virtual size of the X-ray source system 102.

To achieve high resolution, an embodiment of the X-ray micro tomography system 100 further utilizes a very high resolution detector 124-1 of the detector system 118, preferably in conjunction with positioning the sample 114 close to the X-ray source system 102. In one implementation of the high-resolution detector 124-1, a scintillator or other X-ray-to-light conversion device is used in conjunction with possibly a microscope objective to provide additional optical magnification in a range between 2x and 100x, or more.

Other possible detectors or detector-lens combinations can be included as part of the detector system 118 in the illustrated X-ray CT system 100. For example, the detector system 118 can include a lower resolution detector 124-2. This could be a flat panel detector or a detector with a lower magnification microscope objective, in examples. Configurations of one, two, or even more detectors 124 of the detector system 118 are possible.

Preferably, two or more objective lens/detector 124-1, 124-2 are mounted on a rotating turret 122 of the detector system 118, so that they can be alternately rotated into the path of the attenuated beam 106 from the sample 114. In some embodiments one image detector can acquire images from different objective lenses, which are located in the imaging path.

Typically, based on operator defined parameters, the controller 210 (acquisition software) of the computer system 200 sends instructions to the control interface 130, which is usually embedded in programs, to initiate movements of rotation and transfer motors of the rotation stage 110 and detectors to position the sample 114 and the detector 124 for the SRWFM set up. After completion, the controller 210 rotates the sample 114 relative to the beam 104 to perform the CT scan of the sample 114 and saves the projection data 262 to a datastore 260.

In one example, the computer system 200 includes a graphics or other acceleration processor 220 that analyzes the X-ray projections and possibly performs the calculations necessary for tomographic reconstructions represented by volume datasets 264 created from the X-ray projections. A display device 240, connected to the computer system 200, displays information from the X-ray CT system 100. An input device 250 such as a touch screen, keyboard, and/or computer mouse enables interaction between the operator, the computer system 200, and the display device 240.

Using user interface applications executing on the computer system 200 that display their interfaces on the display device 240, in one example, the operator defines/selects CT scan or calibration parameters including whether to employ a SRWFM scan. These include X-ray acceleration voltage settings, and settings for defining the X-ray energy spectrum of the scan and exposure time on the X-ray source system 102. The operator also typically selects other settings such as the number of X-ray projection images to create for the sample 114, and the angles to rotate the rotation stage 110.

The computer system 200, with the assistance of its image processor 220, accepts the image or projection information from the detector system 118 associated with each rotation angle of the sample 114. Often the image processor 220 combines the projection images using reconstruction algorithms to create 3D tomographic reconstructed volume information 264 for the sample 114.

The computer system 200 will typically execute an analytical reconstruction application 254 and/or an iterative reconstruction application 256 for reconstructing the volume data set of the sample 114 from the projection data 262.

The reconstruction applications 254, 256 runs on top of the operating system 252. The operating system 252, in turn, is executed by the computer's central processing unit(s) (CPU) 250.

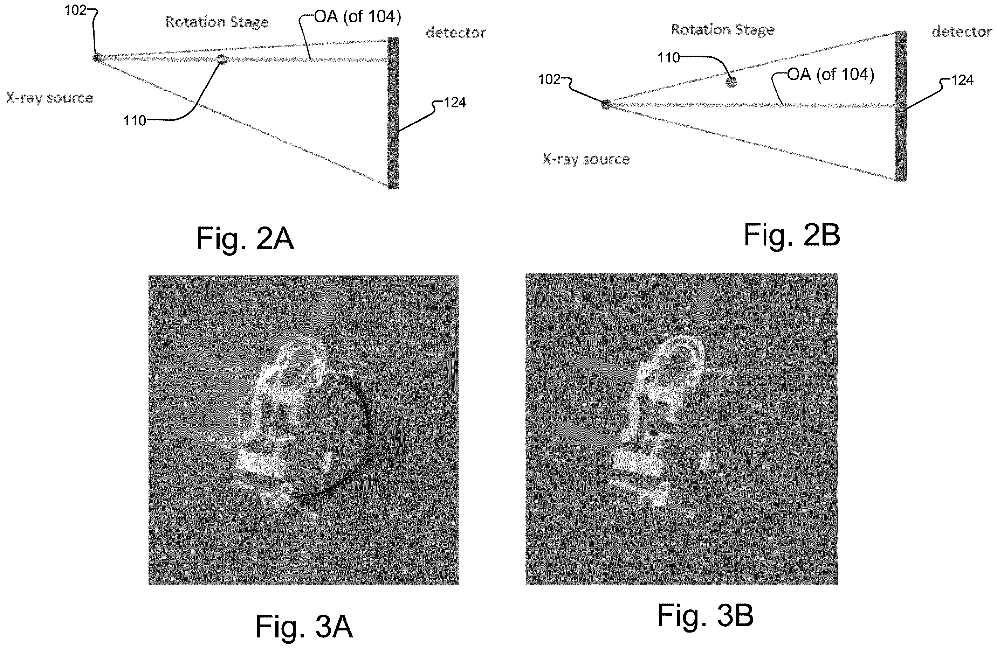

Figs. 2A and 2B are schematic top views of two hardware configurations for single rotation wide field mode scans. The detector 124 is shifted to one side in Fig. 2A with respect to the center or axis OA of the cone formed by the X-ray beam 104. That means, the line passing through the source (point) and perpendicular to the detector is no longer the center or axis of the cone (optical axis) of the X-ray beam. In Fig. 2B, the rotation stage 110 is shifted to one side of the detector with respect to the optical axis OA of the X-ray cone OA 104. That means, the rotation stage 110 is outside of the line passing through the source and perpendicular to the detector 124.

By this shifting, the projections acquired by the detector are truncated with respect to the shifted side, but they are widened with respect to the other side. The truncated part has more or less to be estimated in the following reconstruction.

Both of these configurations increase the field of view of the reconstructed 3D volume that is reconstructed from the truncated projections, which was acquired, using the detector, by shifting the rotation stages, or detectors to one side, and this is defined as single rotation wide field mode (SRWFM).

Figs. 3A and 3B are image artifacts of SRWFM scans with FDK reconstruction results (Fig. 3A) and iterative reconstruction results (Fig. 3B).

Both exhibit artifacts on the reconstructed slices. A ring-shaped image artifact is notable in the FDK reconstruction but less visible in iterative reconstruction compared. Nevertheless, the artifact problem requires further attention.

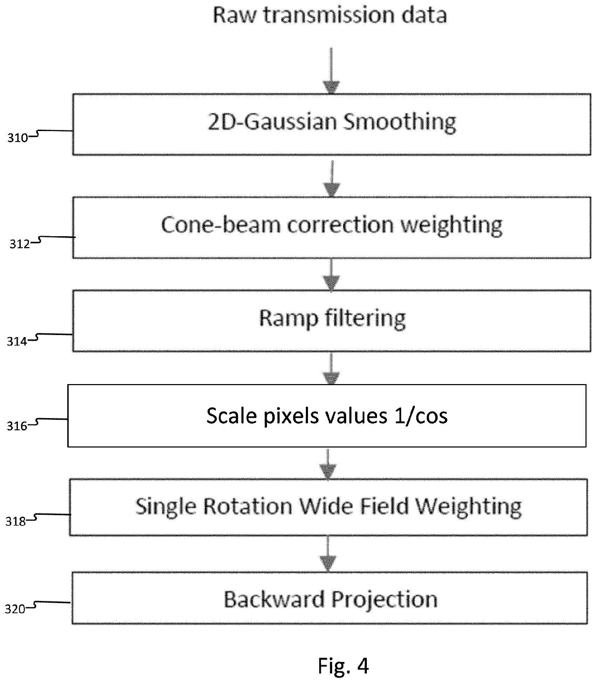

Fig. 4 is a flow diagram showing the steps to process projection data (Raw transmission data) acquired by the image detector 124 using FDK filtered back projection for SRWFM and performed by a computer system such as computer system 200.

In step 310, two-dimensional Gaussian smoothing could be (not necessarily) performed on the projection data. This is useful to improve the Signal-to-Noise Ratio (SNR) in the final reconstructed volumes.

In step 312, cone beam correction weighting, which is a weighting related to the cone-beam CT geometry, is performed on the projections characteristic of FDK reconstruction. In a cone-beam geometry, the density of rays changes as a function of distance from the source. This can lead to varying contributions of different rays to the final reconstruction. Cone-beam weighting functions are used to compensate for this non-uniformity by adjusting the intensity values before back-projection, as described in Feldkamp, L.A., Davis, L.C. and Kress, J.W. (1984) Practical Cone-Beam Algorithm. Journal of the Optical Society of America A, 1, 612-619.

In step 314, ramp filtering is performed on the projections. This serves to counterbalance the blurring introduced by the back-projection process. The back-projection operation is inherently a smoothing process, and without any correction, the reconstructed images would be blurred. This blurring effect arises because each pixel in the reconstructed image is essentially an average of the attenuated line integrals along the rays through that pixel.

In this variant of FDK algorithm, redundancy weighting of the measured data is not performed (Cho, P.S., Rudd, A.D. and Johnson, R.H., Cone-beam CT from width-truncated projections. Computerized Medical Imaging and Graphics, 20, 49-57 (1996)). As we know in mathematics, multiplication of two signals in spatial domain equals a convolution in frequency domain of the two signals. Therefore, pre-weighting the raw measured data is to multiply the raw measured data with the redundancy weighting function, which is a convolution operation in the signal frequency domain. Therefore, this step could introduce extra frequency components, which may be amplified by the ramp filtering step, since the ramp filter is a high pass filter. Instead, the redundancy weighting is performed after convolution with a ramp filter.

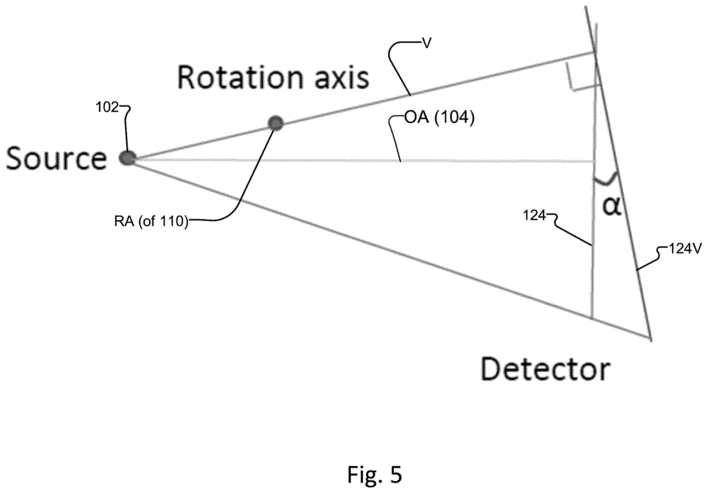

Specifically, in step 316, the pixel intensities are scaled as $1/\cos(\alpha)$, where $\alpha$ is the angle as shown in Fig. 5. That is, $\alpha$ is the angle between the plane of the detector 124 and a virtual detector plane 124V that is normal to a vector V passing through the rotation axis RA of the rotation stage 110 and the center of the source 102. This makes the image intensity scale correct for the rotation axis shifted SRWFM by making the intensity in the reconstructed volume represent the attenuation coefficients of the sample.

Alternatively e.g., raw projection data can be remapped onto a virtual detector plane perpendicular to the line from source 102 to the rotation axis RA of the rotation stage 110 in the principal plane of the cone-beam geometry. Then, the reconstruction is performed using the projections on the virtual detector.

Then, in step 318, Redundancy Weighting (Single Rotation Wide Field Weighting) is performed on the 2D ramp-filtered projection data before backward projection step.

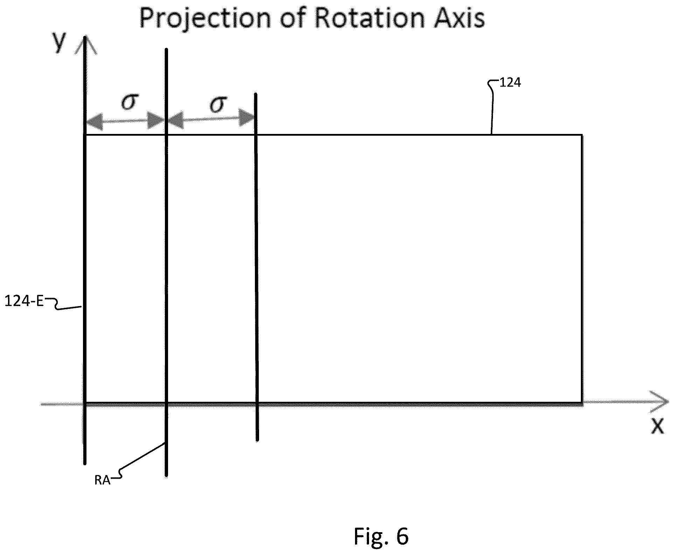

Fig. 6 shows the relationship between the rotation axis RA of the rotation stage 110 with respect to the area of the detector 124 when the location of the rotation stage's axis of rotation RA is projected onto the detector 124 in a SRWFM configuration when looking at the detector 124 from the perspective of the source 110. This shows how the factor $\sigma$ is defined, which is the distance between the rotation axis RA projected onto the detector 124 and the closest edge 124-E of the detector 124 running parallel to the projected rotation axis RA.

Redundancy weighting (SRWF Weighting) of the ramp-filtered 2D raw data of step 318 is performed according to the following weighting formula:

$$w(x) = \begin{cases} 1.0 + \sin\dfrac{\pi x}{4\sigma} & 0 \leq x \leq 2\sigma \\ 2 & otherwise \end{cases} \tag{1}$$

Where $\sigma$ is defined as in Fig. 6.

This formula is employed when the rotation axis of the rotation stage 110 is shifted to the left with respect to the detector center as shown in Fig. 6. If the rotation axis is shifted to the right with respect to the detector, a flipped version of the weighting formula is used, i.e., wherein w(x) takes a value of 2 for each x except for any x that satisfies $x_{max}-2\square \leq x \leq x_{max}$, wherein $x_{max}$ denotes the closest (right) edge of the detector 124 running parallel to the projected rotation axis RA.

Finally, in step 320 of Fig. 4, backward projection is performed, that is, the pre-processed projection data is "smeared back" to each voxel of the reconstructed volume, that is, each pixel value of the pre-processed projection data is put back to the voxels of the reconstructed volume that intersects with the X-ray line path from X-ray source to the detector pixels.

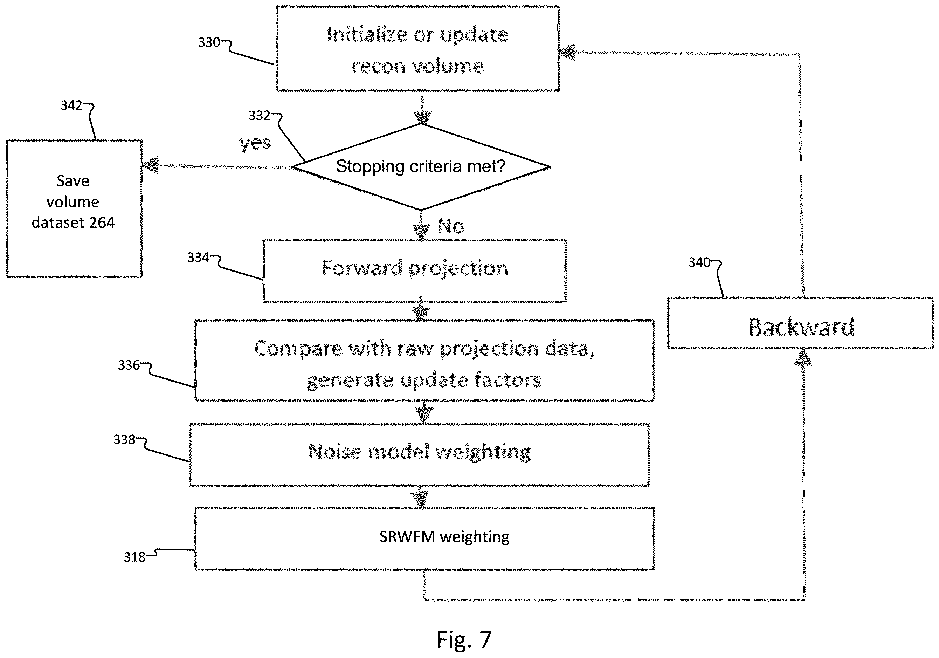

Fig. 7 is a flow diagram showing the method for iterative reconstruction of a SRWFM scan performed by a computer system such as computer system 200.

In step 330, the reconstructed volume is initialized to zero.

The stopping criteria based on the reconstructed volume is checked in step 332. One stopping criteria could be based on a pre-determined number of iterations. If the number of iterations reaches the pre-determined value, then the method stops. Other stopping criteria could be based on the change of the final reconstructed volume, that is, if the voxel value changes are smaller than a predefined value between iterations, then the method stops. In either case, the final reconstruction volume set 264 is saved in step 342.

In step 334, the forward projection is performed.

In step 336, the raw projection is compared with the forward projection from step 334 to compute the differences between the raw projection data and the forward-projected data, which are used to update the reconstruction volume in the back-projection step.

In step 338, the noise model weighting is performed, which adjusts the contribution of each X-ray line integral, measured along the straight line connecting the source and a given detector pixel, to the reconstructed image volume. In more detail, the X-ray line integrals having larger attenuation values and hence larger uncertainty (or noise) should contribute less to the reconstruction volume than those having smaller attenuation values and smaller noise.

Then in step 318, the previously defined Single Rotation Wide Field Weighting is performed as described in connection with Fig. 4.

This improves iterative reconstruction image quality and reduces artifacts by adding the extra SRWFM weighting step using weighting formula in the iterative reconstruction.

The iterative reconstruction proceeds by backward projecting 340 with the weighted projections from step 318 to create the updated reconstructed volume in step 330.

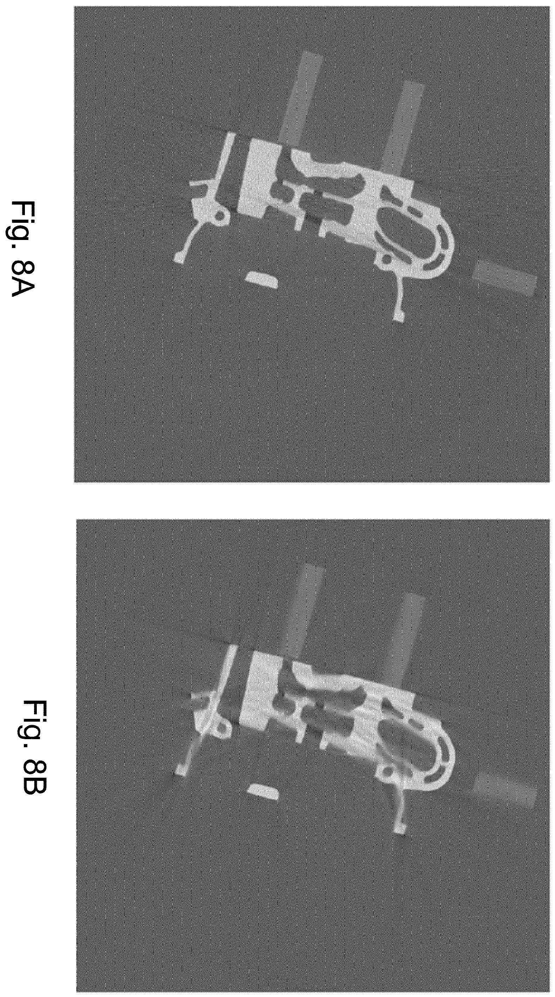

Figs. 8A and 8B show image reconstruction results according FDK-based method of Fig. 4 (Fig. 8A) and according to the iterative reconstruction method of Fig. 7 (Fig. 8B).

In one example of the SRWFM scans for FDK result and iterative reconstruction result, image artifacts for both reconstruction methods are gone using the proposed methods.

While this invention has been particularly shown and described with references to preferred embodiments thereof, it will be understood by those skilled in the art that various changes in form and details may be made therein without departing from the scope of the invention encompassed by the appended claims.

## Claims

1. A method for reconstructing a three-dimensional volume of a sample (114) from projection data obtained by a single rotation wide field mode, SRWFM, X-ray-microscopy tomographic scan, the method comprising:

   acquiring, by a detector (124), X-ray projections of the sample (114) by rotating the sample about a rotation axis, RA, that is offset with respect to an X-ray propagation axis, OA, of an X-ray beam (104) emitted by an X-ray source (102); and

processing the acquired projections to account for the offset rotation axis, RA, by applying redundancy weighting to correct for incomplete angular sampling in regions of the projection data, wherein the redundancy weighting is performed before back-projection in an analytical reconstruction or before an equivalent back-projection operation in an iterative reconstruction.

2. The method of claim 1, further comprising, prior to the redundancy weighting, applying a ramp filter to the projection data, wherein the redundancy weighting is performed on the ramp-filtered data.

3. The method of claim 1 or 2, wherein the redundancy weighting is applied in a region around a projection of the rotation axis, RA, on the detector (124).

4. The method of claim 3, wherein the redundancy weighting employs a position-dependent weighting function.

5. The method of any one of claims 1 to 4, further comprising: adjusting the projection data by scaling pixel intensities according to an angle formed between the propagation axis, OA, of the X-ray beam (104) and a plane of the detector (124) and/or according to an angle formed between a plane of the detector (124 and a virtual detector plane (124V) that is normal to a vector, V, passing through the rotation axis, RA, and the center of the source (102).

6. The method of any one of claims 1 to 4, wherein the projections are remapped onto a virtual detector plane (124V) perpendicular to a line connecting the X-ray source (102) and the rotation axis, RA.

7. The method of any one of claims 1 to 6, wherein the iterative reconstruction approach includes applying a noise model-based weighting to a difference between measured and forward-projected data, and subsequently incorporating the redundancy weighting before back-projection.

8. The method of any one of claims 1 to 7, further comprising acquiring projections over a, preferably over only one, full 360-degree rotation of the sample (114) to ensure sufficient sampling of regions within the enlarged field of view.

9. A system (100) for reconstructing a three-dimensional volume of a sample (114) from projection data obtained by a single rotation wide field mode, SRWFM, X-ray scan, the system comprising:

an X-ray source (102) configured to generate an X-ray beam (104);
a rotation stage (110) configured to hold and rotate the sample (114) about a rotation axis, RA, that is offset with respect to an X-ray propagation axis, OA of the X-ray beam (104), thereby providing X-ray projections with an enlarged effective field of view beyond nominal detector dimensions;
a detector (124) configured to receive the X-ray projections of the sample (114); and a processing system (200) configured to:

receive projection data from the detector (124),
apply redundancy weighting to correct for incomplete angular sampling in regions of the projection data, wherein the redundancy weighting is performed before back-projection in an analytical reconstruction or before an equivalent back-projection operation in an iterative reconstruction, and
reconstruct a three-dimensional volume of the sample (114) from the projection data based on the applied redundancy weighting.

10. The system (100) of claim 9, wherein the processing system (200) is further configured to apply a ramp filter to the projection data prior to applying the redundancy weighting, such that the redundancy weighting is performed on the ramp-filtered data.

11. The system (100) of claim 9 or 10, wherein the processing system (200) is configured to apply the redundancy weighting in a region around a projection of the rotation axis, RA, on the detector (124).

12. The system (100) of claim 11, wherein the processing system (200) is configured to employ a position-dependent weighting function.

13. The system (100) of any one of claims 9 to 12, wherein the processing system (200) is further configured to adjust the projection data by scaling pixel intensities according to an angle formed between the propagation axis, OA, of the X-ray beam (104) and a plane of the detector (124) and/or according to an angle formed between a plane of the detector (124 and a virtual detector plane (124V) that is normal to a vector, V, passing through the rotation axis, RA, and the center of the source (102) or is configured to remap the projections onto the virtual detector plane (124V) perpendicular to a line connecting the X-ray source (102) and the rotation axis, RA.

14. The system (100) of any one of claims 10 to 13, wherein the processing system (200) is configured to execute an iterative reconstruction algorithm that includes applying a noise model-based weighting to differences between measured and forward-projected data, and subsequently incorporating the redundancy weighting before back-projection.

15. The system (100) of any one of claims 10 to 14, wherein the processing system (200) is configured to operate the rotation stage (110) and acquire projections over a, preferably over only one full 360-degree rotation of the sample (114) to ensure sufficient sampling of regions within the enlarged field of view.

Fig. 1

Fig. 2A

Fig. 2B

Fig. 3A

Fig. 3B

Raw transmission data

↑

| 2D-Gaussian Smoothing | ⟋310 |

↑

| Cone-beam correction weighting | ⟋312 |

↑

| Ramp filtering | ⟋314 |

↑

| Scale pixels values 1/cos | ⟋316 |

↑

| Single Rotation Wide Field Weighting | ⟋318 |

↑

| Backward Projection | ⟋320 |

Fig. 4

EP 4 589 539 A1

Fig. 5

Projection of Rotation Axis

Fig. 6

Fig. 7

Fig. 8A

Fig. 8B

Europäisches
Patentamt

European
Patent Office

Office européen
des brevets

# EUROPEAN SEARCH REPORT

Application Number

EP 25 15 1991

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHARMA KRITI SEN ET AL: "Interior micro-CT with an offset detector", MEDICAL PHYSICS, AIP, MELVILLE, NY, US, vol. 41, no. 6, 28 May 2014 (2014-05-28), XP012185941, ISSN: 0094-2405, DOI: 10.1118/1.4876724 [retrieved on 1901-01-01] * page 1 - page 4 * | 1-15 | INV. G06T11/00 |
| A | HANSIS E ET AL: "Iterative reconstruction for circular cone-beam CT with an offset flat-panel detector", NUCLEAR SCIENCE SYMPOSIUM CONFERENCE RECORD (NSS/MIC), 2010 IEEE, IEEE, 30 October 2010 (2010-10-30), pages 2228-2231, XP032054454, DOI: 10.1109/NSSMIC.2010.5874179 ISBN: 978-1-4244-9106-3 * the whole document * | 1-15 | |
| A | WANG GE: "X-ray micro-CT with a displaced detector array", MEDICAL PHYSICS, AIP, MELVILLE, NY, US, vol. 29, no. 7, 1 July 2002 (2002-07-01), pages 1634-1636, XP012011861, ISSN: 0094-2405, DOI: 10.1118/1.1489043 * the whole document * | 1-15 | TECHNICAL FIELDS SEARCHED (IPC) G06T |

The present search report has been drawn up for all claims

| Place of search | Date of completion of the search | Examiner |
|---|---|---|
| Munich | 26 April 2025 | Celik, Hasan |

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FELDKAMP, L.A., DAVIS, L.C. AND KRESS, J.W.** Practical Cone-Beam Algorithm. *Journal of the Optical Society of America A*, 1984, vol. 1, 612-619
- **LIN, Q.** ; **ANDREW, M.** ; **THOMPSON, W.** ; **BLUNT, M.J.** ; **BIJELJIC, B.** Optimization of image quality and acquisition time for lab-based X-ray microtomography using an iterative reconstruction algorithm. *Advances in Water Resources*, 2018, vol. 115, 112-124
- **CHO, P.S.** ; **RUDD, A.D.** ; **JOHNSON, R.H.** Cone-beam CT from width-truncated projections. *Computerized Medical Imaging and Graphics*, 1996, vol. 20, 49-57
- **FELDKAMP, L.A.** ; **DAVIS, L.C.** ; **KRESS, J.W.** Practical Cone-Beam Algorithm. *Journal of the Optical Society of America A*, 1984, vol. 1, 612-619
- **CHO, P.S** ; **RUDD, A.D.** ; **JOHNSON, R.H.** Cone-beam CT from width-truncated projections.. *Computerized Medical Imaging and Graphics*, 1996, vol. 20, 49-57